(19) Europäisches Patentamt
European Patent Office
Office européen des brevets

(11) EP 2 644 683 B1

(12) FASCICULE DE BREVET EUROPEEN

(45) Date de publication et mention de la délivrance du brevet:
**10.08.2022 Bulletin 2022/32**

(21) Numéro de dépôt: **13305267.0**

(22) Date de dépôt: **11.03.2013**

(51) Classification Internationale des Brevets (IPC):
***C10G 45/04*** *(2006.01)* ***C10G 45/06*** *(2006.01)*
***C10G 45/08*** *(2006.01)* ***B01J 21/12*** *(2006.01)*
***C10G 65/04*** *(2006.01)* ***C10G 65/06*** *(2006.01)*

(52) Classification Coopérative des Brevets (CPC):
**B01J 23/28; B01J 23/30; B01J 23/745; B01J 23/75; B01J 23/755; B01J 23/883; B01J 35/1019; B01J 35/1038; B01J 37/0201; B01J 37/20; C10G 29/205; C10G 45/04; C10G 45/06; C10G 45/08; C10G 45/38;** (Cont.)

(54) **Procédé d'hydrogenation selective d'une essence**

Selektives Hydrierverfahren eines Benzins

Method for selective hydrogenation of a gasoline

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **29.03.2012 FR 1200952**

(43) Date de publication de la demande:
**02.10.2013 Bulletin 2013/40**

(73) Titulaire: **IFP Energies nouvelles**
**92500 Rueil-Malmaison (FR)**

(72) Inventeurs:
- **Daudin, Antoine**
  **69960 Corbas (FR)**
- **Devers, Elodie**
  **69007 Lyon (FR)**
- **Gornay, Julien**
  **69520 Grigny (FR)**
- **Leflaive, Philibert**
  **69780 Mions (FR)**

(74) Mandataire: **IFP Energies nouvelles**
**Département Propriété Industrielle**
**Rond Point de l'échangeur de Solaize**
**BP3**
**69360 Solaize (FR)**

(56) Documents cités:
**EP-A2- 0 451 640**
**US-A- 5 597 476**
**US-A- 6 013 598**
**US-A- 6 126 814**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 2 644 683 B1

(52) Classification Coopérative des Brevets (CPC):
(Cont.)
**C10G 45/60; C10G 65/043; C10G 65/06;**
C10G 2300/104; C10G 2300/1088; C10G 2300/202;
C10G 2400/02

## Description

La présente invention concerne un procédé d'hydrogénation sélective d'une essence et un procédé de désulfuration d'essence mettant en œuvre ce procédé d'hydrogénation sélective.

## Etat de la technique

La production d'essences répondant aux nouvelles normes d'environnement nécessite que l'on diminue de façon importante leur teneur en soufre à des valeurs n'excédant généralement pas 50 ppm, et préférentiellement inférieures à 10 ppm.

Il est par ailleurs connu que les essences de conversion, et plus particulièrement celles provenant du craquage catalytique, qui peuvent représenter 30 à 50 % du pool essence, présentent des teneurs élevées en oléfines et en soufre.

Le soufre présent dans les essences est pour cette raison imputable, à près de 90 %, aux essences issues des procédés de craquage catalytique, qu'on appellera dans la suite essence de FCC (Fluid Catalytic Cracking selon la terminologie anglo-saxonne, que l'on peut traduire par craquage catalytique en lit fluidisé). Les essences de FCC constituent donc la charge préférée du procédé de la présente invention.

Parmi les voies possibles pour produire des carburants à faible teneur en soufre, celle qui a été très largement retenue consiste à traiter spécifiquement les bases essences riches en soufre par des procédés d'hydrodésulfuration en présence d'hydrogène. Les procédés traditionnels désulfurent les essences de manière non sélective en hydrogénant une grande partie des mono-oléfines, ce qui engendre une forte perte en indice d'octane et une forte consommation d'hydrogène. Les procédés les plus récents, tels que le procédé Prime G+ (marque commerciale), permettent de désulfurer les essences de craquage riches en oléfines, tout en limitant l'hydrogénation des mono-oléfines et par conséquent la perte d'octane et la forte consommation d'hydrogène qui en résulte. De tels procédés sont par exemples décrits dans les demandes de brevet EP 1077247 et EP 1174485.

Comme décrit dans la demande de brevet EP 1077247, il est avantageux de réaliser avant l'étape d'hydrotraitement une étape d'hydrogénation sélective de la charge à traiter. Cette première étape d'hydrogénation consiste essentiellement à hydrogéner sélectivement les dioléfines, tout en transformant conjointement par alourdissement les composés soufrés légers saturés (par augmentation de leur poids moléculaire), qui sont des composés soufrés dont le point d'ébullition est inférieur au point d'ébullition du thiophène, tels que le méthanethiol, l'éthanethiol, le diméthylsulfure. Cela permet de produire une fraction essence désulfurée composée majoritairement de mono-oléfines à 5 atomes de carbone sans perte d'octane par simple distillation.

Dans des conditions opératoires spécifiques, cette hydrogénation réalise sélectivement l'hydrogénation des dioléfines présentes dans la charge à traiter en composés mono-oléfiniques, qui possèdent un meilleur indice d'octane. Un autre effet de l'hydrogénation sélective est de prévenir la désactivation progressive du catalyseur d'hydrodésulfuration sélective et/ou d'éviter un bouchage progressif du réacteur dû à la formation de gommes de polymérisation à la surface des catalyseurs ou dans le réacteur. En effet, les composés polyinsaturés sont instables et ont tendance à former des précurseurs de gommes de polymérisation.

La demande de brevet EP 2161076 divulgue un procédé d'hydrogénation sélective des composés polyinsaturés, et plus particulièrement des dioléfines, permettant de réaliser conjointement l'alourdissement des composes soufrés légers saturés. Ce procédé met en œuvre un catalyseur contenant au moins un métal du groupe VIb et au moins un métal non noble du groupe VIII déposés sur un support poreux. Cependant ce document ne divulgue pas de catalyseurs permettant de réaliser en outre une isomérisation des oléfines externes en oléfines internes. La présente invention se distingue du catalyseur décrit dans EP 2161076 notamment par la sélection des paramètres suivants pris en combinaison:

- la surface spécifique du catalyseur comprise entre 200 et 270 $m^2/g$,
- la densité de l'élément du groupe VIb exprimée comme étant le rapport entre la teneur en poids d'oxyde de l'élément du groupe VIb et la surface spécifique comprise entre 4 et $6.10^{-4}$ $g/m^2$;
- le rapport molaire entre le métal du groupe VIII et le métal du groupe VIb compris entre 0,6 et 3 mol/mol.

L'étape d'hydrodésulfuration des essences de craquage qui contiennent des mono-oléfines, consiste à faire passer, sur un catalyseur de type sulfure de métal de transition, la charge à traiter mélangée à de l'hydrogène, afin de promouvoir les réactions de réduction du soufre en sulfure d'hydrogène ($H_2S$). Le mélange réactionnel est ensuite refroidi afin de condenser l'essence. La phase gazeuse contenant l'excès d'hydrogène et l'$H_2S$ est séparée et l'essence désulfurée est récupérée.

Les composés soufrés résiduels généralement présents dans l'essence désulfurée peuvent être séparés en deux familles distinctes : les composés soufrés non hydrogénés présents dans la charge d'une part, et les composés soufrés formés dans le réacteur par des réactions secondaires dites de recombinaison. Parmi cette dernière famille de composés soufrés, les composés majoritaires sont les mercaptans issus de l'addition de l'$H_2S$ formé dans le réacteur

sur les mono-oléfines présentes dans la charge. Les mercaptans de formule chimique R-SH où R est un groupement alkyle, sont également appelés mercaptans de recombinaison, et représentent généralement entre 20% poids et 80% poids du soufre résiduel dans les essences désulfurées.

La réduction de la teneur en mercaptans de recombinaison peut être réalisée par hydrodésulfuration catalytique mais au prix d'une saturation d'une partie importante des mono-oléfines présentes dans l'essence, ce qui entraîne alors une forte diminution de l'indice d'octane de l'essence ainsi qu'une surconsommation d'hydrogène. Il est par ailleurs connu que la perte d'octane liée à l'hydrogénation des mono-oléfines lors de l'étape d'hydrodésulfuration est d'autant plus grande que la teneur en soufre visée est basse, c'est à dire que lorsque l'on cherche à éliminer en profondeur les composés soufrés présents dans la charge.

Toba et al. (Applied Catalysis B: Environmental 70 (2007) 542-547*)* et Badawi et al. (Journal of Molecular Catalysis A: Chemical 320 (2010) 34-39*)* ont par ailleurs étudié l'influence de la structure des composés mono-oléfiniques sur leur réactivité dans l'étape d'hydrodésulfuration (HDS) et montré que les composés mono-oléfiniques à double liaison interne étaient plus difficiles à hydrogéner dans les conditions d'hydrodésulfuration.

Le brevet US 5 597 476 décrit un procédé catalytique pour le traitement des essences de FCC.

Afin de produire une essence à basse teneur en soufre et présentant un bon indice d'octane il apparait avantageux de mettre en œuvre une première étape d'hydrogénation sélective qui réalise conjointement l'hydrogénation des dioléfines en oléfines, l'alourdissement des composés soufrés légers et l'isomérisation des oléfines externes en oléfines internes de façon à faciliter le fonctionnement du procédé d'hydrodésulfuration, à limiter autant que possible l'hydrogénation des oléfines et par voie de conséquence limiter la perte d'indice d'octane lors de l'étape d'hydrodésulfuration subséquente.

## Résumé de l'invention

Un but de la présente invention est de proposer un procédé amélioré d'hydrogénation sélective d'une essence contenant des composés polyinsaturés et des composés soufrés légers qui permet une meilleure isomérisation des composés mono insaturés dont la double liaison C=C est externe en double liaison C=C interne tout en assurant l'hydrogénation des composés polyinsaturés en composés mono-insaturés, l'alourdissement des composés soufrés légers saturés par réaction avec les composés insaturés.

Les caractéristique essentielles de la présente invention sont explicitement décrites dans le libellé de la revendication indépendante 1. D'autres caractéristiques de l'invention sont décrites dans les libellés des revendications dépendantes 2 à 10.

L'essence ainsi traitée peut être avantageusement envoyée vers une unité d'hydrodésulfuration catalytique dont des conditions opératoires permettant la transformation des composés organo-soufrés en H2S tout en limitant l'hydrogénation des oléfines.

A cette fin, il est proposé un procédé d'hydrogénation sélective d'une essence comprenant des composés polyinsaturés et des composés soufrés légers, ledit procédé permettant conjointement l'hydrogénation des composés polyinsaturés en composés mono-insaturés, l'alourdissement des composés soufrés légers saturés par réaction avec les composés insaturés et l'isomérisation des composés mono insaturés comportant une double liaison C=C externe en leur isomère à double liaison C=C interne, ladite essence étant une essence du craquage catalytique en lit fluide (FCC) et ayant une température d'ébullition comprise entre 0°C et 280°C, procédé dans lequel on met en contact à une température comprise entre 80°C et 220°C, avec une vitesse spatiale liquide comprise entre $1h^{-1}$ et $10h^{-1}$ et une pression comprise entre 0,5 et 5 MPa, et avec un rapport molaire entre l'hydrogène et les dioléfines à hydrogéner supérieur à 1 et inférieur à 10 mol/mol, l'essence, de l'hydrogène avec un catalyseur sous forme sulfure contenant au moins un métal du groupe VIb et au moins un métal du groupe VIII déposés sur un support poreux, le métal du groupe VIII est le nickel et le métal du groupe VIb est le molybdène, le taux de sulfuration des métaux dudit catalyseur étant supérieur à 80%, et dans lequel :

- la teneur en poids d'oxyde de l'élément du groupe VIb par rapport au poids du catalyseur est comprise entre 6 et 18% ;
- la teneur en poids d'oxyde de l'élément du groupe VIII par rapport au poids du catalyseur est comprise entre 4 et 12% ;
- la surface spécifique du catalyseur est comprise entre 200 et 270 $m^2/g$;
- la densité de l'élément du groupe VIb, exprimée comme étant le rapport entre ladite teneur en poids d'oxyde de l'élément du groupe VIb et la surface spécifique du catalyseur, est comprise entre 4 et $6.10^{-4}$ $g/m^2$;
- le rapport molaire entre le métal du groupe VIII et le métal du groupe VIb est compris entre 0,6 et 3 mol/mol.

De façon surprenante, le demandeur a en effet trouvé qu'en mettant en œuvre un procédé d'hydrogénation en présence d'un catalyseur tel que revendiqué permet de réaliser conjointement l'hydrogénation des composés polyinsaturés et plus particulièrement des dioléfines, l'alourdissement des composés soufrés légers et plus particulièrement des mercaptans et d'améliorer l'isomérisation des composés mono-oléfiniques ayant une double liaison C=C externe

en leurs isomères correspondants à double liaison C=C interne.

Le procédé selon l'invention est applicable à toute coupe essence contenant une certaine proportion de dioléfines, et pouvant contenir en outre quelques composés plus légers appartenant aux coupes C3 et C4.

Un autre but de l'invention est de fournir un procédé de désulfuration d'essence permettant d'obtenir un produit ayant une teneur en S total inférieure à 50 ppm, de préférence inférieure à 10 ppm tout en limitant la perte d'indice d'octane.

A cette fin, il est proposé un procédé de désulfuration comprenant les étapes suivantes:

a) une étape d'hydrogénation sélective mettant en œuvre un procédé décrit précédemment,
b) une étape de séparation de l'essence obtenue à l'étape a) en deux fractions comprenant respectivement une essence légère et une essence lourde.
c) un traitement de l'essence lourde séparée à l'étape b) sur un catalyseur permettant de décomposer au moins partiellement les composés soufrés en $H_2S$

## Description détaillée de l'invention

L'invention concerne un procédé de traitement d'essences comprenant tout type de familles chimiques et notamment des dioléfines, des mono-oléfines, et des composés soufrés sous forme de mercaptans et de sulfures légers. La présente invention trouve particulièrement son application dans la transformation des essences de conversion, et en particulier des essences en provenance du craquage catalytique, du craquage catalytique en lit fluide (FCC), d'un procédé de cokéfaction, d'un procédé de viscoréduction, ou d'un procédé de pyrolyse. Les charges pour lesquelles s'applique l'invention ont une température d'ébullition comprise entre 0°C et 280°C. Les charges peuvent également contenir des hydrocarbures à 3 ou 4 atomes de carbone.

Par exemple, les essences issues d'unités de craquage catalytique (FCC) contiennent, en moyenne, entre 0,5% et 5% poids de dioléfines, entre 20% et 50% poids de mono-oléfines, entre 10 ppm et 0,5% poids de soufre dont généralement moins de 300 ppm de mercaptans. Les mercaptans se concentrent généralement dans les fractions légères de l'essence et plus précisément dans la fraction dont la température d'ébullition est inférieure à 120°C.

Le traitement de l'essence décrit dans le présent procédé d'hydrogénation sélective consiste principalement à :

- hydrogéner sélectivement les dioléfines en mono-oléfines;
- transformer les composés soufrés légers saturés et principalement les mercaptans, en sulfures ou mercaptans plus lourds par réaction avec les mono-oléfines;
- isomériser les composés mono-oléfines ayant leur double liaison C=C externe en leur isomère à double liaison C=C interne.

Les réactions d'hydrogénation des dioléfines en mono-oléfines sont illustrées ci-dessous par la transformation du 1,3 pentadiène, composé instable, qui peut facilement être hydrogéné en pent-2-ène. Toutefois, on cherche à limiter les réactions secondaires d'hydrogénation des mono-oléfines qui dans l'exemple ci-dessous conduiraient à la formation de n-pentane.

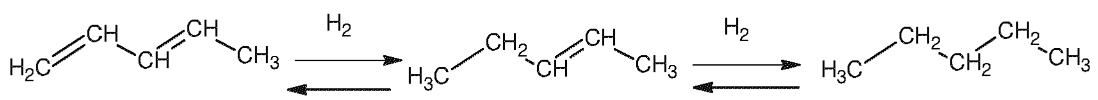


Les composés soufrés que l'on cherche à transformer sont principalement les mercaptans et les sulfures. La réaction principale de transformation des mercaptans consiste en une thioéthérification des mono-oléfines par les mercaptans. Cette réaction est illustrée ci dessous par l'addition du propane-2-thiol sur le pent-2-ène pour former un propylpentyl sulfure.

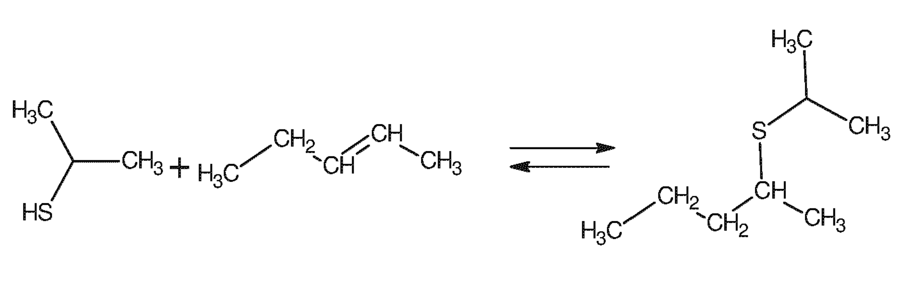

En présence d'hydrogène, la transformation des composés soufrés peut également passer par la formation

intermédiaire d'H2S qui peut ensuite s'additionner sur les composés insaturés présents dans la charge. Cette voie est toutefois minoritaire dans les conditions préférées de la réaction.

Outre les mercaptans, les composés susceptibles d'être ainsi transformés et alourdis sont les sulfures et principalement le diméthyl sulfure, méthyl-éthyl sulfure et diéthyl sulfure, le $CS_2$, le COS, le thiophane, le méthyl-thiophane.

Dans certains cas, on peut également observer des réactions d'alourdissement des composés azotés légers, et principalement des nitriles, du pyrrole et de ses dérivés.

Selon l'invention, le catalyseur permet également de réaliser une isomérisation des composées mono-oléfiniques ayant leur double liaison C=C en position externe en leur isomère ayant leur double liaison C=C en position interne.

Cette réaction est illustrée ci-après par l'isomérisation du hexène-1 en hexène-2 ou hexène-3:

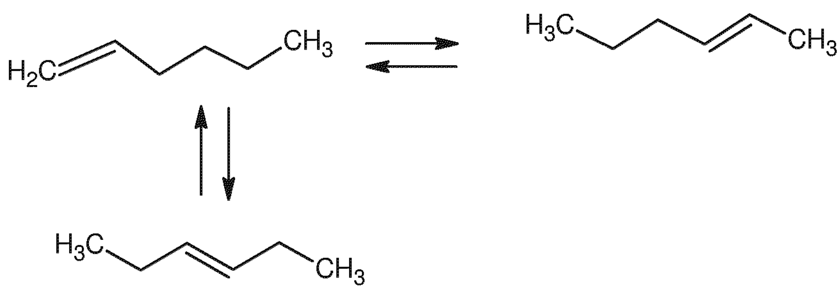

Le procédé décrit dans la présente invention consiste à mettre en contact la charge à traiter en mélange avec un flux d'hydrogène, avec un catalyseur contenant au moins un métal du groupe VIb (groupe 6 selon la nouvelle notation de la classification périodique des éléments : Handbook of Chemistry and Physics, 76ième édition, 1995-1996) et au moins un métal du groupe VIII, (groupes 8, 9 et 10) de ladite classification, déposés sur un support poreux à base d'oxyde métallique.

En particulier, il a été trouvé que les performances des catalyseurs en isomérisation des oléfines externes en oléfines internes correspondants sont améliorées lorsque le catalyseur présente les caractéristiques suivantes en combinaison:

La teneur en poids de l'élément du groupe VIb sous forme oxyde est comprise entre 6 et 18%, de préférence entre 8 et 12% et manière encore préférée entre 10 et 12% en poids par rapport au poids de catalyseur. Le métal du groupe VIb est le molybdène.

Le catalyseur contient également un métal du groupe VIII. Le métal du groupe VIII est le nickel. La teneur en métal du groupe VIII exprimée sous forme d'oxyde est comprise entre 4 et 12% poids et de préférence comprise entre 6 et 10% poids et de manière encore préférée entre 6 et 8% poids par rapport au poids de catalyseur.

Le rapport molaire entre le métal du groupe VIII et le métal du groupe VII , à savoir le rapport molaire entre le Nickel et le Molybdène, est compris entre 0,6 et 3 mol/mol et de manière préférée, entre 1 et 2 mol/mol.

La densité de l'élément du groupe VIb, exprimée comme étant le rapport entre ladite teneur en poids d'oxyde de l'élément du groupe VIb et la surface spécifique du catalyseur, est comprise entre 4 et $6.10^{-4}$ g/$m^2$, de préférence entre 4,3 et $5,5.10^{-4}$ g/$m^2$, de préférence entre 4,5 et $5.10^{-4}$ g/$m^2$. Ainsi par exemple dans le cas de figure où le catalyseur comprend 1 1% poids d'oxyde de molybdène par rapport au poids de catalyseur et présente une surface spécifique de 219 $m^2$/g alors la densité de molybdène, exprimée comme étant le rapport entre la teneur en poids d'oxyde de molybdène et la surface spécifique du catalyseur, est égale à (0,11 / 219) soit $5.10^{-4}$ g/$m^2$.

La surface spécifique du catalyseur est comprise entre 200 et 270 $m^2$/g, de préférence entre 220 et 260 $m^2$/g. La surface spécifique est déterminée selon la norme ASTM D3663.

De préférence, on utilise un catalyseur présentant un volume poreux total mesuré par porosimétrie au mercure supérieur à 0,3 $cm^3$/g, de préférence compris entre 0,4 et 1,4 $cm^3$/g et préférentiellement compris entre 0,5 et 1,3 $cm^3$/g. La porosimétrie au mercure est mesurée selon la norme ASTM D4284-92 avec un angle de mouillage de 140°, avec un appareil modèle Autopore III de la marque Microméritics.

Le support du catalyseur est de préférence choisi parmi l'alumine, l'aluminate de nickel, la silice, le carbure de silicium, ou leur mélange. On utilise, de manière préférée, de l'alumine et de manière encore plus préférée, de l'alumine pure.

Selon une variante, le support est constitué d'alumine gamma cubique ou de l'alumine delta.

Le catalyseur selon l'invention peut être préparé au moyen de toute technique connue de l'homme du métier, et notamment par imprégnation des éléments des groupes VIII et VIb sur le support sélectionné. Cette imprégnation peut par exemple être réalisée selon le mode connu de l'homme du métier sous la terminologie d'imprégnation à sec, dans lequel on introduit juste la quantité d'éléments désirés sous forme de sels solubles dans le solvant choisi, par exemple de l'eau déminéralisée, de façon à remplir aussi exactement que possible la porosité du support. Le support

ainsi rempli par la solution est de préférence séché. Le support préféré est l'alumine qui peut être préparée à partir de tout type de précurseurs et outils de mise en forme connus de l'homme de métier.

Après introduction des éléments des groupes VIII et VIb, , à savoir le Nickel et respectivement le Molybdène, et éventuellement une mise en forme du catalyseur, celui-ci subi un traitement d'activation. Ce traitement a généralement pour but de transformer les précurseurs moléculaires des éléments en phase oxyde. Il s'agit dans ce cas d'un traitement oxydant mais un simple séchage du catalyseur peut également être effectué. Dans le cas d'un traitement oxydant, également appelé calcination, celui-ci est généralement mis en œuvre sous air ou sous oxygène dilué, et la température de traitement est généralement comprise entre 200°C et 550°C, de préférence entre 300°C et 500°C.

Des sels de métaux des groupes VIb et VIII utilisables dans le procédé de préparation du catalyseur sont par exemple le nitrate de nickel, l'heptamolybdate d'ammonium. Tout autre sel connu de l'homme du métier présentant une solubilité suffisante et décomposable lors du traitement d'activation peut également être utilisé.

Après calcination, les métaux déposés sur le support se trouvent sous forme d'oxyde. Dans le cas du nickel et du molybdène, les métaux se trouvent principalement sous forme de $MoO_3$ et de NiO. Avant mise en contact avec la charge à traiter, les catalyseurs subissent une étape de sulfuration. La sulfuration est de préférence réalisée en milieu sulforéducteur, c'est-à-dire en présence d'$H_2S$ et d'hydrogène, afin de transformer les oxydes métalliques en sulfures tels que par exemple, le $MoS_2$ et le $Ni_3S_2$. La sulfuration est réalisée en injectant sur le catalyseur un flux contenant de l'$H_2S$ et de l'hydrogène, ou bien un composé soufré susceptible de se décomposer en $H_2S$ en présence du catalyseur et de l'hydrogène. Les polysulfures tel que le diméthyldisulfure sont des précurseurs d'$H_2S$ couramment utilisés pour sulfurer les catalyseurs. La température est ajustée afin que l'$H_2S$ réagisse avec les oxydes métalliques pour former des sulfures métalliques. Cette sulfuration peut être réalisée in situ ou ex situ (en dedans ou dehors du réacteur) du réacteur d'hydrodésulfuration à des températures comprises entre 200 et 600°C et plus préférentiellement entre 300 et 500°C.

Pour être actifs, les métaux doivent être substantiellement sulfurés. Un élément est considéré comme substantiellement sulfuré lorsque le rapport molaire entre le soufre (S) présent sur le catalyseur et ledit élément est au moins égal à 60% du rapport molaire théorique correspondant à la sulfuration totale de l'élément considéré:

$$(S/\text{élément})\text{catalyseur} \geq 0{,}6 \times (S/\text{élément})\text{théorique}$$

avec:

(S/élément)catalyseur rapport molaire entre le soufre (S) et l'élément présents sur le catalyseur
(S/élément)théorique rapport molaire entre le soufre et l'élément correspondant à la sulfuration totale de l'élément en sulfure.

Ce rapport molaire théorique varie selon l'élément considéré:

$$(S/Fe)\text{théorique} = 1$$

$$(S/Co)\text{théorique} = 8/9$$

$$(S/Ni)\text{théorique} = 2/3$$

$$(S/Mo)\text{théorique} = 2/1$$

$$(S/W)\text{théorique} = 2/1$$

Lorsque le catalyseur comprend plusieurs métaux, le rapport molaire entre le S présent sur le catalyseur et l'ensemble des éléments doit également être au moins égal à 60% du rapport molaire théorique correspondant à la sulfuration totale de chaque élément en sulfure, le calcul étant effectué au prorata des fractions molaires relatives de chaque élément.

Par exemple, pour un catalyseur comprenant du molybdène et du nickel avec une fraction molaire respective de 0,7 et 0,3, le rapport molaire minimal (S/ Mo + Ni) est donné par la relation:

$$(S/Mo+Ni)catalyseur = 0{,}6 \times \{(0{,}7 \times 2)+ (0{,}3 \times (2/3)\}$$

De façon très préférée, le taux de sulfuration des métaux sera supérieur à 80%.

La sulfuration est mise en œuvre sur les métaux sous forme d'oxyde sans que soit réalisée une étape préalable de réduction des métaux. En effet, il est connu que la sulfuration de métaux réduits est plus difficile que la sulfuration de métaux sous forme d'oxydes.

Dans le procédé d'hydrogénation sélective selon l'invention, la charge à traiter est mélangée à de l'hydrogène avant d'être mise en contact avec le catalyseur. La quantité d'hydrogène injectée est telle que le rapport molaire entre l'hydrogène et les dioléfines à hydrogéner soit supérieur à 1 (stœchiométrie) et inférieure à 10, et de préférence compris entre 1 et 5 mol/mol. Un trop large excès d'hydrogène peut entraîner une forte hydrogénation des mono-oléfines et par voie de conséquence, une diminution de l'indice d'octane de l'essence. La totalité de la charge est généralement injectée à l'entrée du réacteur. Toutefois, il peut être avantageux, dans certains cas d'injecter une fraction ou la totalité de la charge entre deux lits catalytiques consécutifs placés dans le réacteur. Ce mode de réalisation permet notamment de continuer à opérer le réacteur si l'entrée du réacteur se trouve bouchée par dépôts de polymères, de particules, ou de gommes présentes dans la charge.

Le mélange constitué de l'essence et de l'hydrogène est mis en contact avec le catalyseur à une température comprise entre 80°C et 220°C, et de préférence entre 90°C et 200°C, avec une vitesse spatiale liquide (LHSV) comprise entre 1 $h^{-1}$ et 10 $h^{-1}$, l'unité de la vitesse spatiale liquide étant le litre de charge par litre de catalyseur et par heure (l/l.h). La pression est ajustée afin que le mélange réactionnel soit majoritairement sous forme liquide dans le réacteur. La pression est comprise entre 0,5 MPa et 5 MPa et de préférence entre 1 et 4 MPa.

L'essence traitée dans les conditions énoncées ci-dessus, présente une teneur en dioléfines et en mercaptans réduite. Généralement, l'essence produite contient moins de 1 % poids de dioléfines, et de préférence moins de 0,5% poids de dioléfines. Les composés soufrés légers dont la température d'ébullition est inférieure à celle du thiophène (84°C) sont généralement convertis à plus de 50%. Il est donc possible de séparer la fraction légère de l'essence par distillation et d'envoyer directement cette fraction au pool essence sans traitement complémentaire. La fraction légère de l'essence a généralement un point final inférieur à 120°C, et de préférence inférieure à 100°C et de façon très préférée inférieure à 80°C.

Le procédé d'hydrogénation sélective selon l'invention est particulièrement adapté pour être mis en œuvre dans le cadre du procédé de désulfuration décrit dans la demande de brevet EP 1 077 247.

La présente demande a également pour objet un procédé de désulfuration d'essence comprenant des composés soufrés, comprenant au moins les étapes suivantes:

a) une étape d'hydrogénation sélective mettant en œuvre le procédé décrit précédemment;
b) une étape de séparation de l'essence obtenue à l'étape a) en deux fractions comprenant respectivement une essence légère et une essence lourde;
c) une étape de traitement de l'essence lourde séparée à l'étape b) sur un catalyseur permettant de décomposer au moins partiellement les composés soufrés en $H_2S$.

L'étape b) de séparation est réalisée de préférence au moyen d'une colonne de distillation classique appelée aussi splitter. Cette colonne de fractionnement doit permettre de séparer une fraction légère de l'essence contenant une faible fraction du soufre et une fraction lourde contenant de préférence la majeure partie du soufre initialement présent dans l'essence initiale.

Cette colonne opère généralement à une pression comprise entre 0,1 et 2 MPa et de préférence entre 0,2 et 1 MPa. Le nombre de plateaux théoriques de cette colonne de séparation est généralement compris entre 10 et 100 et de préférence entre 20 et 60. Le taux de reflux, exprimé comme étant le rapport du débit liquide dans la colonne divisé par le débit de distillat exprimé en kg/h, est généralement inférieur à l'unité et de préférence inférieur à 0,8.

L'essence légère obtenue à l'issue de la séparation contient généralement au moins l'ensemble des oléfines en C5, de préférence les composés en C5 et au moins 20 % des oléfines en C6. Généralement, cette fraction légère présente une faible teneur en soufre, c'est à dire qu'il n'est pas en général nécessaire de traiter la coupe légère avant de l'utiliser comme carburant.

L'étape c) de désulfuration est de préférence une étape d'hydrodésulfuration réalisée par passage de l'essence lourde, en présence d'hydrogène, sur un catalyseur comprenant au moins un élément du groupe VIII et/ou au moins un élément du groupe VIb au moins en partie sous forme sulfure, à une température comprise entre environ 210°C et environ 350°C, de préférence entre 220°C et 320°C, sous une pression généralement comprise entre environ 1 et environ 4 MPa, de préférence entre 1,5 et 3 MPa. La vitesse spatiale du liquide est comprise entre environ 1 et environ 20 $h^{-1}$ (exprimée en volume de liquide par volume de catalyseur et par heure), de préférence entre 1 et 10 $h^{-1}$, de manière très préférée entre 3 et 8 $h^{-1}$. Le rapport $H_2$/charge est compris entre 100 à 600 litres par litre et préférentiellement entre

300 et 600 litres par litre.

En ce qui concerne le catalyseur à utiliser dans l'étape c) ci dessus, la teneur en métal du groupe VIII exprimée en oxyde est généralement comprise entre 0,5 et 15% poids, préférentiellement entre 1 et 10 % poids par rapport au poids du catalyseur. La teneur en métal du groupe VIb exprimée en oxyde est généralement comprise entre 1,5 et 60% poids, préférentiellement entre 3 et 50% poids par rapport au poids de catalyseur.

En ce qui concerne le catalyseur à utiliser dans l'étape c) ci-dessus, l'élément du groupe VIII, lorsqu'il est présent, est de préférence le cobalt, et l'élément du groupe VIb, lorsqu'il est présent, est généralement le molybdène ou le tungstène. Des combinaisons telles que cobalt molybdène sont préférées. Le support du catalyseur est habituellement un solide poreux, tel que par exemple une alumine, une silice-alumine ou d'autres solides poreux, tels que par exemple de la magnésie, de la silice ou de l'oxyde de titane, seuls ou en mélange avec de l'alumine ou de la silice-alumine. Pour minimiser l'hydrogénation des oléfines présentes dans l'essence lourde, il est avantageux d'utiliser préférentiellement un catalyseur dans lequel la densité de molybdène, exprimée en % poids de $MoO_3$ (le % en poids étant exprimé par rapport au poids total du catalyseur) par unité de surface spécifique est supérieure à 0,07 et de préférence supérieure à 0,12. Le catalyseur selon l'invention présente de préférence une surface spécifique inférieure à 250 $m^2$/g, de manière plus préférée inférieure à 230$m^2$/g, et de manière très préférée inférieure à 190 $m^2$/g.

Le dépôt des métaux sur le support est obtenu pour toutes méthodes connues de l'homme de l'art telles que par exemple l'imprégnation à sec, par excès d'une solution contenant les précurseurs de métaux. La solution d'imprégnation est choisie de manière à pouvoir solubiliser les précurseurs de métaux dans les concentrations désirées. Par exemple, dans le cas de la synthèse d'un catalyseur CoMo, le précurseur de molybdène peut être l'oxyde de molybdène, l'heptamolybdate d'ammonium et tandis que le précurseur de cobalt peut être par exemple le nitrate de cobalt, l'hydroxyde de cobalt, le carbonate de cobalt. Les précurseurs sont généralement dissous dans un milieu permettant leur solubilisation dans les concentrations désirées.

Après introduction du ou des éléments et éventuellement mise en forme du catalyseur, le catalyseur est dans une première étape activé. Cette activation peut correspondre soit à une oxydation puis à une réduction, soit à une réduction directe, soit à une calcination uniquement. L'étape de calcination est généralement réalisée à des températures allant d'environ 100 à environ 600°C et de préférence comprises entre 200 et 450°C, sous un débit d'air. L'étape de réduction est réalisée dans des conditions permettant de convertir au moins une partie des formes oxydées du métal de base en métal. Généralement, elle consiste à traiter le catalyseur sous un flux d'hydrogène à une température de préférence au moins égale à 300 °C. La réduction peut aussi être réalisée en partie au moyen de réducteurs chimiques.

Le catalyseur est de préférence utilisé au moins en partie sous sa forme sulfurée. L'introduction du soufre peut intervenir avant ou après toute étape d'activation, c'est-à-dire de calcination ou de réduction. De préférence, aucune étape d'oxydation du catalyseur n'est réalisée lorsque le soufre ou un composé soufré a été introduit sur le catalyseur. Le soufre ou un composé soufré peut être introduit ex situ, c'est-à-dire en dehors du réacteur où le procédé selon l'invention est réalisé, ou in situ, c'est-à-dire dans le réacteur utilisé pour le procédé selon l'invention. Dans ce dernier cas, le catalyseur est de préférence réduit dans les conditions décrites précédemment, puis sulfuré par passage d'une charge contenant au moins un composé soufré, qui une fois décomposé conduit à la fixation de soufre sur le catalyseur. Cette charge peut être gazeuse ou liquide, par exemple de l'hydrogène contenant de l'$H_2S$, ou un liquide contenant au moins un composé soufré.

D'une façon préférée, le composé soufré est ajouté sur le catalyseur ex situ. Par exemple, après l'étape de calcination, un composé soufré peut être introduit sur le catalyseur en présence éventuellement d'un autre composé. Le catalyseur est ensuite séché, puis transféré dans le réacteur servant à mettre en œuvre le procédé selon l'invention. Dans ce réacteur, le catalyseur est alors traité sous hydrogène afin de transformer au moins une partie du métal principal en sulfure. Une procédure qui convient particulièrement à l'invention est celle décrite dans les brevets FR-B- 2 708 596 et FR-B- 2 708 597.

Selon un mode particulier de l'invention, le procédé de désulfuration comprend en outre une deuxième étape d) de traitement de l'essence lourde traitée à l'étape c) sur un catalyseur permettant de décomposer les composés soufrés qui n'auraient pas été décomposés en $H_2S$ lors de l'étape c). De façon avantageuse on procède à l'élimination de l'$H_2S$ formé à l'étape c) avant d'effectuer l'étape de traitement d).

## Exemple 1

Les catalyseurs 1, 2, 3 et 4 ont été préparés par imprégnation à sec d'un support 100% alumine.

Le protocole de synthèse consiste à réaliser une imprégnation à sec d'une solution d'heptamolybdate d'ammonium et de nitrate de nickel, le volume de la solution aqueuse contenant les précurseurs métalliques étant égal au volume de reprise à l'eau correspondant à la masse de support à imprégner (volume d'eau total pouvant pénétrer dans la porosité). Les concentrations des précurseurs dans la solution sont ajustées de manière à déposer sur le support les teneurs pondérales en oxydes métalliques souhaitées.

Le solide est ensuite laissé en maturation à température ambiante durant 12 heures, puis séché à 120°C,

durant 12 heures. Finalement, le solide est calciné à 500°C durant deux heures sous flux d'air (1 L/g.h).

Les supports utilisés sont des alumines de transition à surface spécifique variable, de façon à obtenir des catalyseurs à différentes surface spécifiques après un chargement à iso-teneur en métaux. Les caractéristiques des catalyseurs ainsi préparés sont fournies dans le tableau 1 ci-dessous.

Tableau 1 : Caractéristiques des catalyseurs 1, 2, 3 et 4 sous forme oxyde.

| Catalyseur | 1 (comparatif) | 2 (comparatif) | 3 (mis en œuvre selon l'invention) | 4 (mis en œuvre selon l'invention) |
|---|---|---|---|---|
| % pds en $MoO_3$ | 11 | 11 | 11 | 11 |
| % pds en NiO | 7 | 7 | 7 | 7 |
| Rapport molaire Ni/Mo | 1,2 | 1,2 | 1,2 | 1,2 |
| dMoO3 ($10^{-4}$g/$m^2$ cata) | 8,9 | 5,8 | 4,8 | 4,3 |
| Surface spécifique du catalyseur ($m^2$/g) | 123 | 189 | 230 | 254 |

Les catalyseurs 1 et 2 (non-conformes à l'invention) présentent une surface spécifique en dehors de la gamme 200 - 270 $m^2$/g. En revanche les catalyseurs 3 et 4 ont une surface spécifique et une densité en molybdène conforme à l'invention.

Les performances catalytiques des catalyseurs 1, 2, 3 et 4 ont été évaluées au moyen d'un test d'hydrogénation sélective d'un mélange de molécules modèles effectué dans un réacteur autoclave agité de 500 ml. 4 grammes de catalyseur sont sulfurés à pression atmosphérique en banc de sulfuration sous mélange $H_2S/H_2$ constitué de 15% volumique d'$H_2S$ à 1 L/g.h de catalyseur et à 400°C durant deux heures. Ce protocole permet d'obtenir des taux de sulfuration supérieurs à 80% pour l'ensemble des catalyseurs conformément à l'invention. Le catalyseur ainsi sulfuré est transféré dans le réacteur à l'abri de l'air puis mis au contact de 250 mL de charge modèle sous une pression totale de 1,5 MPa et une température de 160°C. La pression est maintenue constante durant le test par apport d'hydrogène.

La charge utilisée pour le test d'activité présente la composition suivante : 1000 ppm poids de soufre sous forme méthyl 3-thiophène, 100 ppm poids de soufre sous forme de propane-2-thiol, 10% poids oléfine sous forme de d'hexène-1 et 1% poids d'isoprène dans du n-heptane.

Le temps t=0 du test correspond à la mise en contact du catalyseur et de la charge. La durée du test est fixée à 80 minutes et l'analyse chromatographique en phase gaz de l'effluent liquide obtenu permet d'évaluer les activités des différents catalyseurs en hydrogénation de l'isoprène (formation des méthylbutènes), hydrogénation de l'hexène-1 (formation du n-hexane), isomérisation de l'hexène-1 (formation d'hexène avec saturation en position interne, soit hexène-2 ou hexène-3) et alourdissement des mercaptans légers (conversion du propane-2-thiol).

L'activité du catalyseur pour chaque réaction est définie par rapport à la constante de vitesse obtenue pour chaque réaction normalisée par gramme de catalyseur. La constante de vitesse est calculée en considérant un ordre 1 pour la réaction :

$$A(X) = k(X) / m$$

avec : A(X) : activité du catalyseur pour la réaction X, en $min^{-1}$ / g de catalyseur
m : masse de catalyseur (forme oxyde) engagée dans le test
k constante de vitesse pour la réaction considérée, en $min^{-1}$ étant calculée selon la formule:

$$k(X) = (1/80) * \ln (100 / (100 - Conv(X)))$$

avec 80: durée du test en minutes
X = hexène correspondant à l'isomérisation de l'hexène
X = hexène correspondant à l'hydrogénation de l'hexène
X = isoprène correspondant à l'hydrogénation de l'isoprène

La sélectivité du catalyseur vis à vis de l'hydrogénation de l'isoprène est égale au rapport des activités du catalyseur en hydrogénation de l'isoprène et en hydrogénation de l'hexène 1 : A HYD (isoprène)/A HYD (hexène-1)

Les résultats obtenus pour les différents catalyseurs sont reportés dans le tableau 2 ci-dessous sur la base de 100 par rapport au catalyseur 3 selon l'invention.

Tableau 2: Performances des catalyseurs en test molécules modèles

| Catalyseur | 1 (comparatif) | 2 (comparatif) | 3 (selon l'invention) | 4 (selon l'invention) |
|---|---|---|---|---|
| A ISOM hexène rel /g | 58 | 78 | 100 | 109 |
| A HYD hexène rel /g | 78 | 87 | 100 | 101 |
| A HYD isoprène rel /g | 75 | 84 | 100 | 106 |
| A HYD (isoprène)/A HYD (hexène-1) | 96 | 97 | 100 | 105 |

Les catalyseurs 3 et 4 (mis en œuvre dans le procédé selon l'invention) dont la densité en molybdène et la surface spécifique tombent dans les gammes revendiquées montrent non seulement une activité en isomérisation de l'hexène qui est nettement supérieure à celle des catalyseurs 1 et 2 mais également une sélectivité en hydrogénation améliorée.

**Exemple 2**

Les exemples de catalyseurs présentés ci-dessous permettent d'illustrer l'influence de la densité du métal du groupe VIb (ici le molybdène) à iso surface spécifique de catalyseur sur l'activité en hydrogénation des dioléfines en mono-oléfines et en isomérisation des mono-oléfines "externes" en mono-oléfines "internes".

Des catalyseurs 5 et 6 (non conformes à l'invention) ont été préparés selon les conditions de l'exemple 1. Ces catalyseurs possèdent notamment une surface spécifique conforme à l'invention mais une densité de molybdène en dehors de la gamme 4 -6.$10^{-4}$ g/$m^2$. Les caractéristiques de ces deux catalyseurs sont regroupées dans le tableau 3.

Tableau 3 : Caractéristiques des catalyseurs 5 et 6 sous forme oxyde

| Catalyseur | 5 (comparatif) | 6 (comparatif) |
|---|---|---|
| % pds en $MoO_3$ | 6 | 9 |
| % pds en NiO | 3,8 | 5,6 |
| Rapport molaire Ni/Mo | 1,2 | 1,2 |
| dMoO3 ($10^{-4}$g/$m^2$ cata) | 2,4 | 3,8 |
| Surface spécifique du catalyseur ($m^2$/g) | 253 | 239 |

Les performances catalytiques des catalyseurs 5 et 6 sont évaluées dans le test d'hydrogénation sélective décrit dans l'exemple 1 et comparées avec celles des catalyseurs 3 et 4 qui sont conformes à l'invention.

Tableau 4: Performances des catalyseurs en test molécules modèles

| Catalyseur | 5 (comparatif) | 4 (selon l'invention) | 6 (comparatif) | 3 (selon l'invention) |
|---|---|---|---|---|
| A ISOM hexène rel /g | 69 | 109 | 82 | 100 |
| A HYD hexène rel /g | 61 | 101 | 72 | 100 |
| A HYD isoprène rel /g | 59 | 106 | 73 | 100 |
| A HYD (isoprène)/A HYD (hexène-1) | 97 | 105 | 101 | 100 |

En comparant les catalyseurs 5 et 4 et les catalyseurs 6 et 3, on observe qu'à iso surface spécifique (respectivement environ 250 et 230 $m^2$/g), un catalyseur présentant une densité de molybdène qui tombe dans la gamme revendiquée, possède une activité en hydrogénation sélective des dioléfines et en isomérisation qui sont significativement plus élevées par rapport aux catalyseurs 5 et 6 dont la densité en molybdène est situé en dehors de la gamme 4 à 6.$10^{-4}$ g/$m^2$ (respectivement 2,4.$10^{-4}$g/$m^2$ et 3,8.$10^{-4}$g/$m^2$).

**Exemple 3**

Les catalyseurs 7 (non-conforme à l'invention) et 8 (selon l'invention) ont été préparés selon les conditions de l'exemple 1. Ces catalyseurs se distinguent par leur rapport molaire entre le métal du groupe VIII et le métal du groupe VIb.

Tableau 5 : Caractéristiques des catalyseurs 7 et 8 sous forme oxyde

| Catalyseur | 7 (comparatif) | 8 (selon l'invention) |
|---|---|---|
| % pds en $MoO_3$ | 11 | 11 |
| % pds en NiO | 3 | 11 |
| Rapport molaire Ni/Mo | 0,5 | 1,9 |
| dMoO3 ($10^{-4}$g/m$^2$ cata) | 4,5 | 5,0 |
| Surface spécifique du catalyseur (m$^2$/g) | 241 | 219 |

Les catalyseurs 7 et 8 ont été évalués avec le test d'hydrogénation sélective décrit à l'exemple 1. Les performances des catalyseurs sont regroupées dans le tableau 6.

Tableau 6: Performances des catalyseurs en test molécules modèles

| Catalyseur | 7 (comparatif) | 8 (selon l'invention) | 3 (selon l'invention) |
|---|---|---|---|
| A ISOM hexène rel /g | 71 | 112 | 100 |
| A HYD hexène rel /g | 118 | 95 | 100 |
| A HYD isoprène rel /g | 68 | 108 | 100 |
| A HYD (isoprène)/A HYD (hexène-1) | 58 | 114 | 100 |

Le catalyseur 7 (non-conforme), qui possède un rapport molaire Ni/Mo en dehors de la gamme 0,6-3 mol/mol mais une densité en molybdène et une surface spécifique tombant dans les gammes revendiquées, présente une moins bonne sélective en hydrogénation et une plus faible activité en isomérisation que les catalyseurs 8 et 3 dont les rapports molaires Ni/Mo sont respectivement de 1,9 et 1,2, les densités en molybdène sont respectivement de 5,0 et 4,8.$10^{-4}$ g/m$^2$ conforme à la gamme 4 à 6.$10^{-4}$ g/m$^2$ et dont les surfaces spécifiques respectivement de 219 et 230 m$^2$/g tombent dans la gamme 200 - 270 m$^2$/g.

Cet exemple permet de confirmer que l'effet technique recherché est bien obtenu en mettant en œuvre un catalyseur qui satisfait à l'ensemble des paramètres pris en combinaison et dans les gammes spécifiques revendiquées.

## Revendications

1. Procédé d'hydrogénation sélective d'une essence comprenant des composés polyinsaturés et des composés soufrés légers, ledit procédé permettant conjointement l'hydrogénation des composés polyinsaturés en composés mono-insaturés, l'alourdissement des composés soufrés légers saturés par réaction avec les composés insaturés et l'isomérisation des composés mono insaturés comportant une double liaison C=C externe en leur isomère à double liaison C=C interne, ladite essence étant une essence du craquage catalytique en lit fluide (FCC) et ayant une température d'ébullition comprise entre 0°C et 280°C, procédé dans lequel on met en contact à une température comprise entre 80°C et 220°C, avec une vitesse spatiale liquide comprise entre 1 h$^{-1}$ et 10h$^{-1}$ et une pression comprise entre 0,5 et 5 MPa, et avec un rapport molaire entre l'hydrogène et les dioléfines à hydrogéner supérieur à 1 et inférieur à 10 mol/mol, l'essence, de l'hydrogène avec un catalyseur sous forme sulfure contenant au moins un métal du groupe VIb et au moins un métal du groupe VIII déposés sur un support poreux, le métal du groupe VIII est le nickel et le métal du groupe VIb est le molybdène, le taux de sulfuration des métaux dudit catalyseur étant supérieur à 80%, et dans lequel :

    • la teneur en poids d'oxyde de l'élément du groupe VIb par rapport au poids du catalyseur est comprise entre 6 et 18% ;
    • la teneur en poids d'oxyde de l'élément du groupe VIII par rapport au poids du catalyseur est comprise entre 4 et 12% ;

- la surface spécifique du catalyseur est comprise entre 200 et 270 $m^2/g$;
- la densité de l'élément du groupe VIb, exprimée comme étant le rapport entre ladite teneur en poids d'oxyde de l'élément du groupe VIb et la surface spécifique du catalyseur, est comprise entre 4 et $6.10^{-4}$ $g/m^2$;
- le rapport molaire entre le métal du groupe VIII et le métal du groupe VIb est compris entre 0,6 et 3 mol/mol.

**2.** Procédé selon l'une des revendications précédentes dans lequel la teneur en poids d'oxyde de l'élément du groupe VIII est comprise entre 6 et 10%, de préférence entre 6 et 8% par rapport au poids du catalyseur, et dans lequel la teneur en poids d'oxyde de l'élément du groupe VIb est comprise entre 8 et 12%, de préférence entre 10 et 12%, par rapport au poids du catalyseur.

**3.** Procédé selon l'une des revendications précédentes dans lequel le rapport molaire entre le métal du groupe VIII et le métal du groupe VIb est compris entre 1 et 2 mol/mol.

**4.** Procédé selon l'une des revendications précédentes dans lequel la densité de l'élément du groupe VIb est comprise entre 4,3 et $5,5.10^{-4}$ $g/m^2$, de préférence entre 4,5 et $5.10^{-4}$ $g/m^2$.

**5.** Procédé selon l'une des revendications précédentes dans lequel le catalyseur présente un volume poreux total supérieur à 0,3 $cm^3/g$.

**6.** Procédé selon l'une des revendications précédentes dans lequel la surface spécifique du catalyseur est comprise entre 220 et 260 $m^2/g$.

**7.** Procédé selon l'une des revendications précédentes dans lequel le support est choisi parmi l'alumine, la silice, le carbure de silicium ou leur mélange.

**8.** Procédé de désulfuration d'essence comprenant des composés soufrés comprenant les étapes suivantes :

a) une étape d'hydrogénation sélective mettant en œuvre un procédé selon l'une des revendications 1 à 7;
b) une étape de séparation de l'essence obtenue à l'étape a) en deux fractions comprenant respectivement une essence légère et une essence lourde;
c) un traitement de l'essence lourde séparée à l'étape b) sur un catalyseur permettant de décomposer au moins partiellement les composés soufrés en $H_2S$.

**9.** Procédé selon la revendication 8, dans lequel l'étape c) est réalisée en présence d'hydrogène, sur un catalyseur comprenant au moins un élément du groupe VIII et/ou au moins un élément du groupe VIb au moins en partie sous forme sulfure, à une température comprise entre 210°C et 350°C, à une pression comprise entre 1 et 4 MPa, avec une vitesse spatiale exprimée en volume de liquide par volume de catalyseur et par heure comprise entre 1 $h^{-1}$ et 20 $h^{-1}$.

**10.** Procédé selon l'une des revendications 8 ou 9, dans lequel le catalyseur de l'étape c) comprend une teneur en métal du groupe VIII exprimée en oxyde comprise entre 0,5 et 15% poids, préférentiellement entre 1 et 10% poids par rapport au poids du catalyseur et une teneur en métal du groupe VIb exprimée en oxyde comprise entre 1,5 et 60% poids, préférentiellement entre 3 et 50% poids par rapport au poids de catalyseur.

## Patentansprüche

**1.** Verfahren zur selektiven Hydrierung eines Benzins, das mehrfach ungesättigte Verbindungen und leichte Schwefelverbindungen umfasst, wobei das Verfahren gleichzeitig die Hydrierung der mehrfach ungesättigten Verbindungen zu einfach ungesättigten Verbindungen, das Schwerermachen der gesättigten leichten Schwefelverbindungen durch Reaktion mit den ungesättigten Verbindungen und die Isomerisierung der einfach ungesättigten Verbindungen mit einer externen C=C-Doppelbindung zu ihrem Isomer mit interner C=C-Doppelbindung ermöglicht, wobei es sich bei dem Benzin um ein Benzin aus dem katalytischen Cracken in der Wirbelschicht (FCC) mit einer Siedetemperatur zwischen 0 °C und 280 °C handelt, wobei man bei einer Temperatur zwischen 80 °C und 220 °C mit einer Flüssigkeitsbelastung zwischen 1 $h^{-1}$ und 10 $h^{-1}$ und einem Druck zwischen 0,5 und 5 MPa und mit einem Molverhältnis zwischen Wasserstoff und zu hydrierenden Diolefinen von mehr als 1 und weniger als 10 mol/mol das Benzin und Wasserstoff mit einem Katalysator in Sulfidform, der mindestens ein Metall der Gruppe VIb und mindestens ein Metall der Gruppe VIII, die auf einem porösen Träger abgeschieden sind, enthält, in Kontakt bringt, wobei es sich

bei dem Metall der Gruppe VIII um Nickel handelt und es sich bei dem Metall der Gruppe VIb um Molybdän handelt, wobei der Sulfurierungsgrad der Metalle des Katalysators über 80 % liegt und wobei:

• der Gewichtsgehalt an Oxid des Elements der Gruppe VIb, bezogen auf das Gewicht des Katalysators, zwischen 6 und 18 % liegt;
• der Gewichtsgehalt an Oxid des Elements der Gruppe VIII, bezogen auf das Gewicht des Katalysators, zwischen 4 und 12 % liegt;
• die spezifische Oberfläche des Katalysators zwischen 200 und 270 $m^2/g$ liegt;
• die Dichte des Elements der Gruppe VIb, ausgedrückt als das Verhältnis zwischen dem Gewichtsgehalt des Oxids des Elements der Gruppe VIb und der spezifischen Oberfläche des Katalysators, zwischen 4 und $6.10^{-4}$ $g/m^2$ liegt;
• das Molverhältnis zwischen dem Metall der Gruppe VIII und dem Metall der Gruppe VIb zwischen 0,6 und 3 mol/mol liegt.

**2.** Verfahren nach einem der vorhergehenden Ansprüche, wobei der Gewichtsgehalt an Oxid des Elements der Gruppe VIII zwischen 6 und 10 %, vorzugsweise zwischen 6 und 8 %, bezogen auf das Gewicht des Katalysators, liegt und wobei der Gewichtsgehalt an Oxid des Elements der Gruppe VIb zwischen 8 und 12 %, vorzugsweise zwischen 10 und 12 %, bezogen auf das Gewicht des Katalysators, liegt.

**3.** Verfahren nach einem der vorhergehenden Ansprüche, wobei das Molverhältnis zwischen dem Metall der Gruppe VIII und dem Metall der Gruppe VIb zwischen 1 und 2 mol/mol liegt.

**4.** Verfahren nach einem der vorhergehenden Ansprüche, wobei die Dichte des Elements der Gruppe VIb zwischen 4,3 und $5,5.10^{-4}$ $g/m^2$, vorzugsweise zwischen 4,5 und $5.10^{-4}$ $g/m^2$, liegt.

**5.** Verfahren nach einem der vorhergehenden Ansprüche, wobei der Katalysator ein Gesamtporenvolumen von mehr als 0,3 $cm^3/g$ aufweist.

**6.** Verfahren nach einem der vorhergehenden Ansprüche, wobei die spezifische Oberfläche des Katalysators zwischen 220 und 260 $m^2/g$ liegt.

**7.** Verfahren nach einem der vorhergehenden Ansprüche, wobei der Träger aus Aluminiumoxid, Siliciumdioxid, Siliciumcarbid oder einer Mischung davon ausgewählt ist.

**8.** Verfahren zur Entschwefelung von Benzin, das Schwefelverbindungen umfasst, umfassend die folgenden Schritte:

a) einen Schritt der selektiven Hydrierung durch Anwendung eines Verfahrens nach einem der Ansprüche 1 bis 7;
b) einen Schritt der Trennung des in Schritt a) erhaltenen Benzins in zwei Fraktionen, die ein leichtes Benzin bzw. ein schweres Benzin umfassen,
c) eine Behandlung des in Schritt b) abgetrennten schweren Benzins an einem Katalysator, der die zumindest teilweise Zersetzung der Schwefelverbindungen zu $H_2S$ erlaubt.

**9.** Verfahren nach Anspruch 8, wobei Schritt c) in Gegenwart von Wasserstoff an einem Katalysator, der mindestens ein Element der Gruppe VIII und/oder mindestens ein Element der Gruppe VIb zumindest teilweise in Sulfidform umfasst, bei einer Temperatur zwischen 210 °C und 350 °C, bei einem Druck zwischen 1 und 4 mPa mit einer als Flüssigkeitsvolumen pro Katalysatorvolumen und pro Stunde ausgedrückten Belastung zwischen 1 $h^{-1}$ und 20 $h^{-1}$ durchgeführt wird.

**10.** Verfahren nach Anspruch 8 oder 9, wobei der Katalysator in Schritt c) einen Gehalt an Metall der Gruppe VIII, ausgedrückt als Oxid, zwischen 0,5 und 15 Gew.-%, vorzugsweise zwischen 1 und 10 Gew.-%, bezogen auf das Gewicht des Katalysators, und einen Gehalt an Metall der Gruppe VIb, ausgedrückt als Oxid, zwischen 1,5 und 60 Gew.-%, vorzugsweise zwischen 3 und 50 Gew.-%, bezogen auf das Gewicht des Katalysators, aufweist.

## Claims

**1.** Process for the selective hydrogenation of a gasoline comprising polyunsaturated compounds and light sulfur compounds, said process allowing concomitantly the hydrogenation of the polyunsaturated compounds to monounsaturated compounds, the increase in molecular weight of the saturated light sulfur compounds through reaction with the unsaturated compounds, and the isomerization of the monounsaturated compounds having an external C=C double bond to the internal C=C double bond isomer thereof, said gasoline being a gasoline from fluid catalytic cracking (FCC) and having a boiling temperature between 0°C and 280°C, process wherein at a temperature of between 80°C and 220°C, a liquid hourly space velocity of between 1 $h^{-1}$ and 10 $h^{-1}$ and a pressure of between 0.5 and 5 MPa, and with a molar ratio between hydrogen and the diolefins to be hydrogenated of greater than 1 and less than 10 mol/mol, the gasoline and hydrogen are contacted with a catalyst in the form of sulfide containing at least one group VIb metal and at least one group VIII metal deposited on a porous support, the group VIII metal is nickel and the group VIb metal is molybdenum, the degree of sulfidation of the metals in said catalyst being greater than 80%, and wherein

- the content by weight of the oxide of the group VIb element relative to the weight of the catalyst is between 6 and 18%,
- the content by weight of the oxide of the group VIII element relative to the weight of the catalyst is between 4 and 12%,
- the specific surface area of the catalyst is between 200 and 270 $m^2/g$,
- the density of the group VIb element, expressed as being the ratio between said content by weight of the oxide of the group VIb element and the specific surface area of the catalyst, is between 4 and $6 \times 10^{-4}$ $g/m^2$,
- the molar ratio between the group VIII metal and the group VIb metal is between 0.6 and 3 mol/mol.

**2.** Process according to one of the preceding claims, wherein the content by weight of the oxide of the group VIII element is between 6 and 10%, preferably between 6 and 8%, relative to the weight of the catalyst, and wherein the content by weight of the oxide of the group VIb element is between 8 and 12%, preferably between 10 and 12%, relative to the weight of the catalyst.

**3.** Process according to either of the preceding claims, wherein the molar ratio between the group VIII metal and the group VIb metal is between 1 and 2 mol/mol.

**4.** Process according to one of the preceding claims, wherein the density of the group VIb element is between 4.3 and $5.5 \times 10^{-4}$ $g/m^2$, preferably between 4.5 and $5 \times 10^{-4}$ $g/m^2$.

**5.** Process according to one of the preceding claims, wherein the catalyst has a total pore volume of greater than 0.3 $cm^3/g$.

**6.** Process according to one of the preceding claims, wherein the specific surface area of the catalyst is between 220 and 260 $m^2/g$.

**7.** Process according to one of the preceding claims, wherein the support is selected from alumina, silica, silicon carbide or a mixture thereof.

**8.** Process for the desulfurization of gasoline comprising sulfur compounds, comprising the following steps:

a) a step of selectively hydrogenating by means of a process according to one of Claims 1 to 7,
b) a step of separating the gasoline obtained in step a) into two fractions comprising respectively a light gasoline and a heavy gasoline,
c) treating the heavy gasoline separated in step b) on a catalyst allowing the at least partial decomposition of the sulfur compounds to $H_2S$.

**9.** Process according to Claim 8, wherein step c) is carried out in the presence of hydrogen, on a catalyst comprising at least one group VIII element and/or at least one group VIb element at least partially in the form of sulfide, at a temperature of between 210°C and 350°C, at a pressure of between 1 and 4 MPa, with a space velocity expressed as the volume of liquid per volume of catalyst per hour of between 1 $h^{-1}$ and 20 $h^{-1}$.

**10.** Process according to either of Claims 8 and 9, wherein the catalyst in step c) has a content of group VIII metal expressed as the oxide of between 0.5% and 15% by weight, preferably between 1% and 10% by weight, relative to the weight of the catalyst, and a content of group VIb metal expressed as the oxide of between 1.5% and 60% by weight, preferably between 3% and 50% by weight, relative to the weight of the catalyst.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- EP 1077247 A
- EP 1174485 A
- EP 2161076 A
- US 5597476 A
- FR 2708596 B
- FR 2708597 B


### Littérature non-brevet citée dans la description


- **TOBA et al.** *Applied Catalysis B: Environmental,* 2007, vol. 70, 542-547
- **BADAWI et al.** *Journal of Molecular Catalysis A: Chemical,* 2010, vol. 320, 34-39
- Handbook of Chemistry and Physics. 1995